# EUROPEAN PATENT SPECIFICATION

(11) **EP 1 773 870 B1**
(45) Date of publication and mention of the grant of the patent: **16.12.2009**
(21) Application number: 06759010.9
(22) Date of filing: 02.05.2006
(51) Int. Cl.: C07K 14/575

(54) **METHODS FOR THE PRODUCTION OF PEPTIDE HAVING A C-TERMINAL AMIDE**
VERFAHREN ZUR HERSTELLUNG VON PEPTIDDERIVATEN
MÉTHODE D'OBTENTION DE DÉRIVÉS PEPTIDIQUES

(30) Priority: 03.05.2005 US 677582 P
(43) Date of publication of application: 18.04.2007
(60) Divisional application: 09010808.5
(73) Proprietor: Novetide, Ltd., 26111 Haifa Bay (IL)
(72) Inventor: IVCHENKO, Alexander, Keryat Ata 28076 (IL); ALON, Hagi, D.N. 25125 (IL); ELSTER, Shai, Haifa 32811 (IL); SHUSHAN, Shimon, Karmiel, 21926 (IL); EIDELMAN, Chaim, 25167 Oshrat (IL); TOVI, Avi, Zurit 20104 (IL); GADI, Tehila, Kfar Vradim 25147 (IL); BAR-OZ, Leah, 27070 Keryat Bialik (IL); ALTERMAN, Eleonora, 34760 Haifa (IL); ZAOVI, Gil, 27070 Keryat Bialik (IL); BUTILCA, Gabriel-Marcus, Karmiel 20100 (IL)
(74) Representative: Gallagher, Kirk James
(86) International application number: PCT/US2006/017044
(87) International publication number: WO 2006/119388

(56) References cited:
- EP-A- 1 179 537
- EP-A- 1 207 167
- WO-A-94/01451
- WO-A-97/48726
- WO-A-03/093301
- WO-A-03/093302

## Description

### Field of the Invention

The present invention relates to a method of preparing a peptide which is a C-terminal amide derivative and to products thereof.

### Background of the Invention

Peptide synthesis may be either solid-phase synthesis (SPPS) or solution-phase synthesis and generally proceeds from the C-terminus to N-terminus. There are several groups of peptide and peptidomimetic compounds characterized by derivatization at the carboxy terminus of the peptide chain.

The synthesis of derivatized peptides is usually done by a solid phase peptide synthesis (SPPS) or a solution phase synthesis. The SPPS usually involves the use of a resin on which the derivatized peptide is built on. The solution phase synthesis is usually based on fragment condensation.

The SPPS is described in BE 841180 and U.S. Patent No. 4,002,738. By this procedure, proline carrying as a blocking group the t-butyloxy-carbonyl substituent (Boc-) on the amino group is esterified by combination with a chloromethylated divinylbenzene-styrene copolymer (Merrifield resin), using the method described by Stewart, et al. in "SOLID PHASE PEPTIDE SYNTHESIS", (published in 1969 by Freeman & Company). The synthesis is continued sequentially in an automatic synthesizer, applying Boc chemistry for the synthesis of the desired nonapeptide. Deprotection of the Boc group was effected by 4N hydrochloric acid/dioxane. Coupling was achieved by the use of dicyclohexylcarbodiimide in dichloromethane at a 2.9 fold excess. Finally, the peptide resin obtained by this procedure was suspended in 200 ml of 5% triethylamine/methanol and 100 ml of distilled ethylamine was added thereto. After 24 hours, the resin was removed by filtration and the solution evaporated to yield a solid. The solid was dissolved in glacial acetic acid and purified on a silica gel column to obtain tri-protected nonapeptide (with protective groups at Ser, Tyr, and Arg). Final deprotection was done by anhydrous hydrogen fluoride. The crude product was finally purified by chromatography on a Sephadex G-25 column (marketed by Pharmacia of Uppsala, Sweden).

Another example of SPPS is found in U.S. Patent No. 4,005,063.

In general, the peptides can be made by using the SPPS described by Merrifield in J. Am. Chem. Soc., 85, 2149 (1963). More particularly, N-blocked proline is esterified to a chloromethylated divinylbenzene-styrene copolymer. After deblocking, N^{γ}-blocked arginine carrying a labile protective group on the imino-N is coupled to the now free imino group of the proline ester and, after deblocking, this sequence of coupling and deblocking steps is repeated with other amino acids in the sequence of the desired peptide. All of the amino acids are used in their L-form except for the amino acid identified as D-amino acid in the formula. After all of these amino acids are linked in the above sequence with the arginine, tyrosine, serine and optionally the histidine carrying protecting groups, the nonapeptide is removed from the resin via transesterification/ammonolyzis whereby the resin link is replaced by the ethylamide terminus. Subsequent treatment in known fashion removes all the protective groups, producing the peptide in substantially pure form and acceptable yield.

European Patent Application EP 0518656A2 describes a SPPS of the Goserelin sequence on a resin through a linkage which is labile to hydrazine. Cleavage of the peptide from the resin results in the hydrazide derivative, which can be converted into the aza-Gly terminal residue. The protection of side chains is achieved by use of the following protecting groups: BrZ for Tyr, Fmoc for His, and tBu for D-Ser at the 6 position, avoiding protection of the Ser at position 4.

Another European Patent Application, EP 0518655A2, describes a SPPS starting with a resin preloaded with the AzaGly building unit. No protection for the Tyr and Ser side chains at the 4 position is used. The final peptide is treated with hydrazine to hydrolyze possible side products with acylated amino-acid side chains which are incorporated in free form.

European Patent Application EP 1179537, describes a SPPS of a peptide sequence which is carried out sequentially using super acid-labile protecting groups and another type of super acid labile resin in such a way that the peptide could be removed from the resin while keeping side chain protecting groups that can be removed later by another acidic treatment. The C-terminal group such as aza-glycine or ethylamine is attached to the protected peptide chain via a regular amide formation procedure. The main disadvantage of this method is the necessity of applying unique and expensive protection strategies.

Another methodology is a solution phase synthesis, based on fragment condensation, as described by International Patent Publication WO 99/07874. By this method, the required peptide can be obtained by reacting a peptide fragment represented by the following general formula pGlu-His-Trp-OR₁ (wherein R₁ represents lower alkyl) with another peptide fragment represented by the following general formula H-Ser-Tyr-X-Leu-Arg-Pro-Y in the presence of chymotrypsin or a chymotrypsin-like enzyme.

Another variation of the fragment condensation method is disclosed in U.S. Patent No. 4,008,209. In this patent, a nonapeptide amide derivative is produced by a method in which a reagent (A) -- L-pyroglutamic acid or a peptide fragment which has an L-pyroglutamic acid unit (i.e., (Pyr)Glu-) at its N-terminal end and at the same time which, from thereon, comprises the desired amino acid sequence -- is condensed with a reagent (B) -- an amine component which corresponds to the balance of the nonapeptide amide derivative --, the two reagents (A) and (B) being optionally protected by a protecting group or groups, and then the protecting group or groups, if any, are removed.

In another example of the same solution phase synthetic approach (Russian Patent Application RU 2074191) the synthesis is performed according to a 2+(2+(4+1)] fragmentation scheme, applying Cbz chemistry and a side chain unprotected Arg residue.

In another example (International Patent Publication WO 97/48726), the peptide chain is built by a 2+4+3 fragment coupling strategy. Cbz protecting chemistry is applied and one of the intermediates is purified by crystallization, while the final peptide is purified by ion exchange chromatography.

U.S. Patent No. 4,100,274 describes a method of obtaining Goserelin by means of the condensation of three pre-formed fragments which contain -NO₂ as the protecting group for arginine and -Bzl as the protecting group for tyrosine, both of which are labile to hydrogenolysis. In this method, the azaglycine residue is introduced into the C-terminal tripeptide, which is then coupled to Z-Tyr(Bzl)-D-Ser(tBu)-Leu-N₃, to give a fragment which, once the Z group is removed, couples to Pyr-His-Trp-Ser-N₃ to give Goserelin. This last reaction is carried out with all the side chains unprotected with the exception of that belonging to D-Ser(tBu).

### Summary of the Invention

In one embodiment, the present invention provides a method of preparing exenatide comprising: providing amino acid, protected or non-protected, attached in its C-terminal to a super-acid labile resin; coupling said amino acid, with another amino acid, protected or non-protected, in the presence of a coupling reagent; repeating the coupling step to obtain a peptide, wherein the peptide is protected with at least one protecting group which remains on the peptide upon its cleavage from the resin; cleaving said protected peptide from the resin by admixing with a mild acidic solution; and amidating the obtained protected peptide with a suitable amine.

In yet another embodiment, the present invention provides Exenatide acetate, having a purity of at least about 99.0% as determined by HPLC method.

In one embodiment, the present invention provides a pharmaceutical composition comprising exanatide acetate made by one of the processes of the present invention and at least one pharmaceutically acceptable excipient.

In another embodiment, the present invention provides a process for preparing a pharmaceutical formulation comprising combining exanatide acetate made by one of the processes of the present invention, with at least one pharmaceutically acceptable excipient.

In yet another embodiment, the present invention provides the use of exanatide acetate made by one of the processes of the present invention for the manufacture of a pharmaceutical composition.

In one embodiment, the present invention provides a process for preparing exanatide acetate comprising obtaining exanatide according to the process of the present invention, and converting the obtained exanatide to Exenatide acetate

### Detailed Description of the Invention

As used herein, the term "ACN" refers to acetonitrile.

As used herein, the term "Boc" refers to t-Butyloxycarbonyl.

As used herein, the term "Bzl" refers to benzyl.

As used herein, the term "Cbz" refers to benzyloxycarbonyl.

As used herein, the term "DCM" refers to dichloromethane.

As used herein, the term "DIEA" refers to diisopropylethylamine.

As used herein, the term "DMF" refers to dimethylformamide.

As used herein, the term "EDT" refers to ethanedithiol.

As used herein, the term "Fmoc" refers to 9-fluorenylmethoxycarbonyl.

As used herein, the term "HBTU" refers to 2-(1H-Benzotriazole-1-yl)-1,1,3,3-tetramethyluronium hexafluorophosphate.

As used herein, the term "HOBt" refers to N-hydroxybenzotriazole.

As used herein, the term "Pbf" refers to pentamethyldihydrobenzofuransulfonyl.

As used herein, the term "SPPS" refers to solid phase peptide synthesis.

As used herein, the term "TBTU" refers to 2-(1H-Benzotriazole-1-yl)-1,1;3,3-tetramethyluronium tetrafluoroborate

As used herein, the term "tBu" refers to tert-butyl.

As used herein, the term "TFA" refers to trifluoroacetic acid.

As used herein, the term "TIS" refers to triisopropylsilane.

As used herein, the term "Trt" refers to trityl.

As used herein, the term "RT" or "room temperature" refers to a temperature of about 18-25°C, preferably about 20-22°C.

As used herein, the term "mild acidic solution" refers to a solution comprising an acid in an inert organic solvent, in a concentration such that during the cleavage of the peptide from the resin the protecting groups remain the peptide.

As used herein, the term "coupling reagent" refers to any product that activates the carboxyl group of the protected peptide fragment.

The invention relates a method for preparing exenatide that comprises a combination of a solid-phase synthesis (SPPS), using a resin as a solid support, to obtain a protected peptide with a carboxylic C-terminus, and a solution-phase synthesis for the amidation of the C-terminus. The invention further relates to protected peptide precursors and to peptide acetates having a purity of at least about 99.0% as determined by HPLC method.

All stages in the process of the present invention are performed under mild conditions, and thus providing a low content of by-products, a high yield and high purity of the final product. In addition, the processes of the present invention require regular commercially available protected amino acids.

The present invention provides a method of preparing exenatide comprising: providing amino acid, protected or non-protected, attached in its C-terminal to a super-acid labile resin; coupling said amino acid, with another amino acid, protected or non-protected, in the presence of a coupling reagent; repeating the coupling step to obtain a peptide, wherein the peptide is protected with at least one protecting group which remains on the peptide upon its cleavage from the resin; cleaving said protected peptide from the resin by admixing with a mild acidic solution; and amidating the obtained protected peptide with a suitable amine.

The amidation step comprises adding a base. Preferably, the base is diisopropylethylamine.

The super-acid labile resin is selected from the group consisting of: chlorotrityl resin, Rink acid resin, NovaSyn TGT resin, and HMPB-AM resin.

The coupling reagent is 2-(1H-benzotriazole-1-yl)-1,1,3,3-tetramethyluronium tetrafluoroborate (TBTU).

The mild acidic solution is a solution comprising about 0.1% to about 5% of TFA in an organic inert solvent or a mixture of acetic acid with trifluoroethanol and DCM.

Prior to the amidation the protected peptide is isolated. Preferably, the isolation is by precipitation, crystallization, extraction, or chromatography. More preferably, the isolation is by precipitation.

The peptide obtained after the cleavage from the resin is protected Exenatide precursor consisting on amino acids having the sequence of: Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-OH (SEQ. ID. NO. 31). Preferably, the amidation comprises treating protected Exenatide precursor with a coupling reagent in the presence of ammonia in DMF, to obtain protected Exenatide consisting on amino acids having the sequence of Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-NH₂ (SEQ. ID. NO. 31). Preferably, after the amidation, the process further comprises: reacting the protected Exenatide with a an acid composition comprising a TFA solution containing water, TIS and EDT; adding ether to obtain a precipitate of Exenatide consisting on amino acids having the sequence of: H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asp-Gly-Gly-Pro-Ser-Ser-Gly-Ala-Pro-Pro-Pro-Ser-NH₂ (SEQ. ID. NO. 31); and isolating the Exenatide.

The isolation of the peptides is by precipitation.

The precipitation is from a solvent selected from the group consisting of: methyl-tert-butyl ether (MTBE), diethyl ether, diisopropylether and mixtures thereof. Preferably, the solvent is mixed with methanol, ethanol or acetonitrile.

The present invention provides exenatide acetate having a purity of at least about 99.0% as determined by HPLC method.

The present invention provides a pharmaceutical composition comprising exenatide acetate made by one of the processes of the present invention and at least one pharmaceutically acceptable excipient.

The present invention provides a process for preparing a pharmaceutical formulation comprising combining exenatide acetate made by one of the processes of the present invention, with at least one pharmaceutically acceptable excipient.

The present invention provides the use of exenatide acetate made by one of the processes of the present invention for the manufacture of a pharmaceutical, composition.

The present invention provides a process for preparing exenatide acetate comprising obtaining exenatide according to the process of the present invention, to exenatide and converting the obtained exenatide acetate

The present invention provides a process for preparing a pharmaceutical formulation comprising combining the exenatide acetate obtained according to the processes of the present invention with at least one pharmaceutically acceptable excipient.

Methods of administration of a pharmaceutical composition of the present invention can be administered in various preparations depending on the age, sex, and symptoms of the patient. The pharmaceutical compositions can be administered, for example, as tablets, pills, powders, liquids, suspensions, emulsions, granules, capsules, suppositories, injection preparations (solutions and suspensions), and the like.

Pharmaceutical compositions of the present invention can optionally be mixed with exanatide acetate obtained in the present invention and other active ingredients. In addition, pharmaceutical compositions of the present invention can contain inactive ingredients such as diluents, carriers, fillers, bulking agents, binders, disintegrants, disintegration inhibitors, absorption accelerators, wetting agents, lubricants, glidants, surface active agents, flavoring agents, and the like.

Diluents increase the bulk of a solid pharmaceutical composition, and may make a pharmaceutical dosage form containing the composition easier for the patient and care giver to handle. Diluents for solid compositions include, for example, microcrystalline cellulose (e.g. Avicel^{®}, microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit®, potassium chloride, powdered cellulose, sodium chloride, sorbitol and talc.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

Glidants can be added to improve the flowability of a non-compacted solid composition and to improve the accuracy of dosing. Excipients that may function as glidants include colloidal silicon dioxide, magnesium trisilicate, powdered cellulose, starch, talc and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol and tartaric acid.

Solid and liquid compositions may also be dyed using any pharmaceutically acceptable colorant to improve their appearance and/or facilitate patient identification of the product and unit dosage level.

In liquid pharmaceutical compositions of the present invention, the peptide acetate and any other solid excipients are dissolved or suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar may be added to improve the taste.

Preservatives and chelating agents such as alcohol, sodium benzoate, butylated hydroxy toluene, butylated hydroxyanisole and ethylenediamine tetraacetic acid may be added at levels safe for ingestion to improve storage stability.

According to the present invention, a liquid composition may also contain a buffer such as glucomic acid, lactic acid, citric acid or acetic acid, sodium guconate, sodium lactate, sodium citrate or sodium acetate. Selection of excipients and the amounts used may be readily determined by the formulation scientist based upon experience and consideration of standard procedures and reference works in the field.

When preparing injectable (parenteral) pharmaceutical compositions, solutions and suspensions are sterilized and are preferably made isotonic to blood. Injection preparations may use carriers commonly known in the art. For example, carriers for injectable preparations include, but are not limited to, water, ethyl alcohol, propylene glycol, ethoxylated isostearyl alcohol, polyoxylated isostearyl alcohol, and fatty acid esters of polyoxyethylene sorbitan. One of ordinary skill in the art can easily determine with little or no experimentation the amount of sodium chloride, glucose, or glycerin necessary to make the injectable preparation isotonic. Additional ingredients, such as dissolving agents, buffer agents, and analgesic agents may be added.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable administration in any given case will depend on the nature and severity of the condition being treated, the most preferred route of the present invention is oral. The dosages may be conveniently presented in unit dosage form and prepared by any of the methods well-known in the pharmaceutical arts.

Dosage forms include solid dosage forms like tablets, powders, capsules, suppositories, sachets, troches and losenges, as well as liquid syrups, suspensions and elixirs.

The dosage form of the present invention may be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

The active ingredient and excipients may be formulated into compositions and dosage forms according to methods known in the art.

A composition for tableting or capsule filling may be prepared by wet granulation. In wet granulation, some or all of the active ingredients and excipients in powder form are blended and then further mixed in the presence of a liquid, typically water, that causes the powders to clump into granules. The granulate is screened and/or milled, dried and then screened and/or milled to the desired particle size. The granulate may then be tabletted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step. The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable route in any given case will depend on the nature and severity of the condition being treated, the most preferred route of the present invention is oral. The dosages can be conveniently presented in unit dosage form and prepared by any of the methods well-known in the pharmaceutical arts.

While the present invention is described with respect to particular examples and preferred embodiments, it is understood that the present invention is not limited to these examples and embodiments. The present invention as claimed therefore includes variations from the particular examples and preferred embodiments described herein, as will be apparent to one of skill in the art.

### Instruments

### HPLC

The following HPLC methods are according to the European Pharmacopia:

### Example 1 Preparation of Boc-His(Trt)-Gly-Gly-Glu(tBu)-Phe-Thr(tBu)-Ser(tBuu)-Asp(tBu)-Leu-Ser(tBu)-Lys(Doc)-Gln(Trt)-Met-Glu(tBu)-GlutBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-OH (SEO. ID NO. 31) (Exenatide precursor)

Synthesis of the peptide is carried out by a regular stepwise Fmoc SPPS procedure starting from 2-Cl-Trt-chloride resin. The first amino acid (Fmoc-Ser(tBu)) is loaded on the resin as described in previous examples to obtain a loading of about 0.7 mmol/g of amino acid/resin. After washing of the resin and removal of the Fmoc group by treatment with piperidine/DMF, the second amino acid (Fmoc-Pro) is introduced to continue sequence elongation. Fmoc protected amino acids are activated in situ using TBTU/HOBt and subsequently coupled to the resin over about 50 minutes. Diisopropylethylamine or collidine is used during coupling as an organic base. Completion of the coupling is indicated by ninhydrin test. After washing of the resin, the Fmoc protecting group on the α-amine is removed with 20% piperidine in DMF for 20 min. These steps are repeated each time with another amino acid according to the peptide sequence. All amino acids used are Fmoc-Nα protected except the last amino acid, which is protected with Boc. Trifunctional amino acids are side chain protected as follows: Ser(tBu), Thr(tBu), Glu(tBu), Gln(Trt), His(Trt), Arg(Pbf), and Asp(tBu). Three equivalents of the activated amino acids are used in the coupling reactions. At the end of the synthesis, the peptide-resin is washed with DMF, followed by DCM, and dried under vacuum to obtain dry peptide-resin.

The peptide, prepared as described above, is cleaved from the resin at RT using a 1% TFA in DCM solution by three repeated washings (15 min each). The acidic peptide solution is neutralized by DIPEA. The product is precipitated by the addition of 10 volumes of water, filtered and dried in vacuum to obtain crude peptide powder.

### Example 2 H-His-Gly-!51u-Gly-Thr-Phe-Tgr-Ser-Asp-Leu-Ser-Lys-Gln-Met-Glu-Glu-Glu-Ala- Val-Arg-Leu-Phe-Ile-Glu-Trp-Leu-Lys-Asp-Gly-Gly-Pro-Ser-Ser-Gly-Ala-Pro-Pro-Pro-Ser-NH₂ (SEO. ID. NO. 31) (Exenatide)

Crude peptide (prepared as described in Example 1) is reacted with ammonia dissolved in DMF. The activation of the carboxyl group of the peptide is done in-situ by addition of TBTU/HOBt to the reaction mixture. Diisopropylethyl amine is used as an organic base. Completion of the reaction is monitored by HPLC analysis. At the end of the reaction, the DMF solution is added slowly to water and crude protected peptide is precipitated as an off-white solid. The peptide is separated by filtration and dried.

The protected peptide above is treated with a cocktail containing 95% TFA, 2.5% TIS, 2.5% EDT for 2 hours at room temperature. The product is precipitated by the addition of 10 volumes of ether, filtered, and dried in vacuum to obtain crude peptide. Crude peptide is purified on a preparative C18 RP-HPLC column to obtain fractions containing peptide solution at a purity of >98.5%. After exchange of the counterion with acetate (on RP-HPLC), the fractions are collected and lyophilized to obtain final dry peptide, >99.0% pure.

## Claims

1. A method of preparing exenatide, comprising:
a) providing amino acid, protected or non-protected, attached in its C-terminal to a super-acid labile resin;
b) coupling said amino acid, with another amino acid, protected or non-protected, in the presence of a coupling reagent;
c) repeating step b) to obtain a peptide, wherein the peptide is protected with at least one protecting group which remains on the peptide upon its cleavage from the resin;
d) cleaving said protected peptide from the resin by admixing with a mild acidic solution; and
e) amidating the protected peptide obtained in step d) with a suitable amine.

2. The process of claim 1, wherein the amidation in step e) is in the presence of a base, preferably diisopropylethylamine.

3. The process of any preceding claim, wherein the super-acid labile resin is selected from the group consisting of: chlorotrityl resin, Rink acid resin, NovaSyn TGT resin, and HMPB-AM resin.

4. The process of any preceding claim, wherein the coupling reagent is 2-(1H-benzotriazole-1-yl)-1,1,3,3-tetramethyluronium tetrafluoroborate (TBTU).

5. The process of any preceding claim, wherein the mild acidic solution in step d) is a solution comprising about 0.1% to about 5% of TFA in an organic inert solvent or a mixture of acetic acid with trifluoroethanol and DCM:

6. The process of any preceding claim, wherein the protected peptide with the resin obtained in step d) is isolated prior to step e).

7. The process of claim 6, wherein the isolation is by precipitation, crystallization, extraction, or chromatography, preferably the isolation is by precipitation.

8. The process of claim 1, wherein the protected peptide obtained after cleavage from the resin is a protected exenatide precursor consisting of amino acids having the sequence of Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-OH (SEQ. ID. NO. 31).

9. The process of claim 8, wherein the amidation comprises treating a protected exenatide precursor with a coupling reagent in the presence of ammonia in DMF, to obtain a protected exenatide precursor consisting of amino acids having the sequence of Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-NH₂ (SEQ. ID. NO. 31).

10. The process of claim 9, which further comprises, after amidation, reacting the protected exenatide with a an acid composition comprising a TFA solution containing water, TIS and EDT; adding ether to obtain a precipitate of Exenatide consisting of amino acids having the sequence of: H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asp-Gly-Gly-Pro-Ser-Ser-Gly-Ala-Pro-Pro-Pro-Ser-NH₂ (SEQ. ID. NO. 31); and isolating the exenatide.

11. A process for preparing exenatide acetate comprising obtaining exenatide according to any one of claims 8 to 10, and converting the obtained exenatide to exenatide acetate.

12. A pharmaceutical composition comprising exenatide acetate prepared according to claim 11 and at least one pharmaceutically acceptable excipient.

13. Exenatide acetate having a purity of at least about 99.0% as determined by HPLC method.

## Patentansprüche

1. Verfahren zum Herstellen von Exenatide, welches folgendes umfasst:
a) Bereitstellen einer geschützten oder nicht geschützten Aminosäure, die an ihrem C-Ende an ein supersäurelabiles Harz gehänge ist
b) Koppeln der Aminosäure mit einer anderen geschützten oder nicht geschützten Aminosäure in Gegenwart eines Kopplungsreagenz,
c) Wiederholen von Stufe b) unter Erhalt eines Peptids, wobei das Peptid mit wenigstens einer Schutzgruppe geschützt ist, die an dem Peptid bis zu seiner Abspaltung von dem Harz verbleibt,
d) Abspalten des geschützten Peptids von dem Harz durch eine schwach saure Lösung und
e) Amidieren des in Stufe d) erhaltenen geschützten Peptids mit einem geeignethen Kamin.

2. Verfahren gemäß Anspruch 1, wobei die Amidierung in Stufe e) in Gegenwart einer Base, bevorzugt die Diisopropylethylamin verläufe

3. Verfahren gemäß einem der vorangehenden Anspruche, wobei das supersäurelabile Harz ausgewählt ist aus der Gruppe, bestehend aus Chlortrityl-Harz, Trink-Säure-Harz, NovaSyn TGT-Harz und HMPB-AM-Harz.

4. Verfahren gemäß einem der vorangehenden Ansprüche, wobei das Kopplungsreagenz 2-(1H-Benzotriazol-1-yl)-1,1,3,3-tetramethyluronium-tefrafluoroborat (TBTU) ist.

5. Verfahren gemäß einem der vorangehenden Ansprüche, wobei die schwach saure Lösung in Stufe d) eine Lösung ist, die ungefähr 0,1 % bis ungefähr 5 % TFA in einem organisch inerten Lösungsmittel enthält oder eine Mischung von Essigsäure mit Trifluorethanol und DCM.

6. Verfahren gemäß einem der vorangehenden Ansprüche, wobei das in Stufe d) erhaltende geschützte Peptid mit dem Harz vor Stufe e) isoliert wird.

7. Verfahren gemäß Anspruch 6, wobei das Isolieren durch Präzipitation, Kristallisation, Extraktion oder Chromatographie, vorzugsweise durch Präzipitation, erfolgt.

8. Verfahren gemäß Anspruch 1, wobei das nach Spaltung von dem Harz erhaltene geschützte Peptid ein geschützter Exenatid-Vorläufer ist, der Aminosäuren mit der folgenden Sequenz aufweist: Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-OH (SEQ. ID. NO. 31).

9. Verfahren gemäß Anspruch 8, wobei das Amidieren Behandeln eines geschützten Exenatid-Vorläufers mit einem Kopplungsreagenz in Gegenwart von Ammoniak in DMF, umfasst, unter Erhalt eines geschützten Exenatid-Vorläufers, der Aminosäuren mit der folgenden Sequenz aufweist: Boc-His(Trt)-Gly-Glu(tBu)-Gly-Thr(tβu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-NH₂ (SEQ. ID. NO. 31).

10. Verfahren gemäß Anspruch 9, welches weiter nach der Amidierung Umsetzen des geschützten Exenatids mit einer sauren Zusammensetzung umfasst, die eine Wasser, TIS und EDT enthaltende TFA-Lösung umfasst, Zugeben von Ether unter Erhalt eines Präzipitats von Exenatid, welches aus Aminosäuren mit der folgenden Sequenz besteht: H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asp-Gly-Gly-Pro-Ser-Ser-Gly-Ala-Pro-Pro-Pro-Ser-NH₂ (SEQ. ID. NO. 31), und Isolieren des Exenatids.

11. Verfahren zur Herstellung von Exenatidacetat, welches Erhalten von Exenatid gemäß einem der Ansprüche 8 bis 10 umfasst und Umwandeln des erhaltenen Exenatids in Exenatidacetat.

12. Pharmazeutische Zusammensetzung, die gemäß Anspruch 11 hergestelltes Exenatidacetat und wenigstens ein pharmazeutisch verträgliches Bindemittel umfasst.

13. Exenatidacetat mit einer durch ein HPLC-Verfahren bestimmten Reinheit von wenigstens ungefähr 99,0 %.

## Revendications

1. Procédé pour préparer un exénatide, comprenant les étapes suivantes :
a) la mise à disposition d'un acide aminé, protégé ou non protégé, attaché au niveau de son extrémité C-terminale à une résine labile superacide ;
b) le couplage dudit acide aminé à un autre acide aminé, protégé ou non protégé, en présence d'un réactif de couplage ;
c) la répétition de l'étape b) pour obtenir un peptide, où le peptide est protégé avec au moins un groupe protecteur qui reste sur le peptide lors de son clivage de la résine ;
d) le clivage dudit peptide protégé de la résine par mélange avec une solution légèrement acide ; et
e) la réalisation d'une amidation du peptide protégé obtenu à l'étape d) avec une amine appropriée.

2. Procédé selon la revendication 1, où l'amidation à l'étape e) est réalisée en présence d'une base, de préférence la diisopropyléthylamine.

3. Procédé selon l'une quelconque des revendications précédentes, où la résine labile superacide est sélectionnée dans le groupe consistant en : résine chlorotrityle, résine acide Rink, résine NovaSyn TGT, et une résine HMPB-AM.

4. Procédé selon l'une quelconque des revendications précédentes, où le réactif de couplage est du tétrafluoroborate de 2-(1H-benzotriazole-1-yl)-1,1,3,3-tétraméthyluronium (TBTU).

5. Procédé selon l'une quelconque des revendications précédentes, où la solution légèrement acide à l'étape d) est une solution comprenant environ 0,1 % à environ 5 % de TFA dans un solvant inerte organique ou un mélange d'acide acétique avec du trifluoroéthanol et du DCM.

6. Procédé selon l'une quelconque des revendications précédentes, où le peptide protégé avec la résine obtenu à l'étape d) est isolé avant l'étape e).

7. Procédé selon la revendication 6, où l'isolation est réalisée par précipitation, cristallisation, extraction, ou chromatographie, l'isolation étant de préférence réalisée par précipitation.

8. Procédé selon la revendication 1, où le peptide protégé obtenu après le clivage de la résine est un précurseur d'exénatide protégé consistant en des acides aminés ayant la séquence : Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-OH (SEQ ID NO: 31).

9. Procédé selon la revendication 8, où l'amidation consiste à traiter un précurseur d'exénatide protégé avec un réactif de couplage en présence d'ammoniac dans du DMF, afin d'obtenir un précurseur d'exénatide protégé consistant en des acides aminés ayant la séquence de : Boc-His(Trt)-Gly-Glu(tBu)-Gly-Thr(tBu)-Phe-Thr(tBu)-Ser(tBu)-Asp(tBu)-Leu-Ser(tBu)-Lys(Boc)-Gln(Trt)-Met-Glu(tBu)-Glu(tBu)-Glu(tBu)-Ala-Val-Arg(Pbf)-Leu-Phe-Ile-Glu(tBu)-Trp-Leu-Lys(Boc)-Asp(tBu)-Gly-Gly-Pro-Ser(tBu)-Ser(tBu)-Gly-Ala-Pro-Pro-Pro-Ser(tBu)-NH2 (SEQ ID NO: 31).

10. Procédé selon la revendication 9, qui consiste en outre, après l'amidation, à faire réagir l'exénatide protégé avec une composition acide comprenant une solution de TFA contenant de l'eau, du TIS et de l'EDT ; à ajouter de l'éther pour obtenir un précipité d'exénatide consistant en des acides aminés ayant la séquence de : H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asp-Gly-Gly-Pro-Ser-Ser-Gly-Ala-Pro-Pro-Pro-Ser-NH₂ (SEQ ID NO: 31) ; et à isoler l'exénatide.

11. Procédé pour préparer de l'acétate d'exénatide comprenant l'obtention de l'exénatide selon l'une quelconque des revendications 8 à 10, et la conversion de l'exénatide obtenu en acétate d'exénatide.

12. Composition pharmaceutique comprenant de l'acétate d'exénatide préparé selon la revendication 11 et au moins un excipient pharmaceutiquement acceptable.

13. Acétate d'exénatide ayant une pureté d'au moins environ 99,0 %, tel que déterminé par le procédé d'HPLC.
